# EUROPEAN PATENT SPECIFICATION

(11) **EP 0 944 401 B1**
(45) Date of publication and mention of the grant of the patent: **25.04.2001**
(21) Application number: 97951249.8
(22) Date of filing: 20.11.1997
(51) Int. Cl.: A61L 9/12

(54) **CONTAINER FOR EMANATING VOLATILE SUBSTANCES**
BEHÄLTER ZUM ABGEBEN FLÜCHTIGER SUBSTANZEN
RECIPIENT POUR EMANATIONS DE SUBSTANCES VOLATILES

(30) Priority: 27.11.1996 IT PD960288
(43) Date of publication of application: 29.09.1999
(73) Proprietor: BP Europack S.P.A., 36030 Lugo di Vicenza (IT)
(72) Inventor: VISONA', Sergio, I-36010 Vicenza (IT)
(74) Representative: Modiano, Guido, Dr.-Ing.
(86) International application number: EP9706477
(87) International publication number: WO9823304

(56) References cited:
- EP-A- 0 300 286
- WO-A-84/02654
- GB-A- 1 572 603
- US-A- 4 145 001
- US-A- 4 161 283
- US-A- 5 518 790

## Description

### Technical Field

The present invention relates to a container for emanating volatile substances.

### Background Art

The container according to the present invention is of the kind that contains aromatic substances and is closed with a multi-ply film in which two groups of detachable plies can be identified.

Multiple outer plies constitute a barrier for the aromatic substance, while a single inner ply, placed so as to close the containment compartment, is permeable to the volatile substance.

In order to emanate the substance, the entire set of barrier plies is removed and only the permeable ply remains on the container.

Several problems arise in these containers.

On the one hand, when the container is in store (storage before use), the contained substance must not be able to escape in any way; on the other hand, separation of the barrier ply must occur without damaging the permeable ply.

Several kinds of container, manufactured in order to achieve these purposes, are known.

In a conventional solution, the permeable ply is constituted by paper, but this solution has the drawback that the substance permeates the paper and escapes through the edges even while the barrier ply is still coupled (i.e., before use).

This entails the drawback that the containers empty themselves even without being used.

Containers are also known wherein there is provided a film made of a foamed material opening along its thickness when the multi-ply layer that constitutes the barrier is removed.

In this case, the foamed ply does not break uniformly and therefore the emanation of the contained product is unpredictable and therefore uncontrollable.

A solution is also known in which the plies that separate are obtained by coextruding polyethylene (or other permeable resins) with polypropylene or polypropylene copolymers, or nylon and polyethylene.

This coextrusion operation, however, is very critical, since the strength of the connection between the two plies that must subsequently separate is highly influenced by the extrusion temperature.

The document US-A-5 518 790 describes a container for volatile substances, closed by multiple plies, consisting of polyamide and EVOH as outer barrier separable from a gas-permeable barrier consisting of foamed polypropylene together with polyethylene.

### Disclosure of the Invention

The aim of the present invention is to overcome the problems found in conventional containers.

An important object is to provide a container which before separating the barrier ply is impermeable to the volatile product, so that it can be stored even for very long periods.

Another important object is to achieve cohesion between the plies of the container in a manner which can be scarcely affected by the process temperature.

Another object is to provide a container by using conventional components and conventional production methods.

This aim, these objects and others which will become apparent hereinafter are achieved by a container for emanating volatile substances, closed by a film formed by multiple plies including at least one barrier ply for the volatile substance and at least one ply permeable to the volatile substance, characterized in that said film comprises:
- an outer group of plies comprising at least one volatile substance barrier ply; and
- an inner coextruded element coupled on one side to said outer group of plies and on another side to said container, said coextruded element being composed of at least three plies which are, sequentially, a first polyethylene ply, an EVOH ply and a second polyethylene ply, wherein said EVOH ply is detachably coupled to said second polyethylene ply by means of a peelable resin interposed between said EVOH ply and said second polyethylene ply, said second polyethylene ply acting as said at least one volatile substance permeable ply.

EVOH is an ethylene-vinyl alcohol copolymer.

### Brief Description of the Drawings

Further characteristics and advantages of the present invention will become apparent from the following detailed description of an embodiment, which is given only by way of non-limitative example and is illustrated with the aid of the accompanying drawings, wherein:

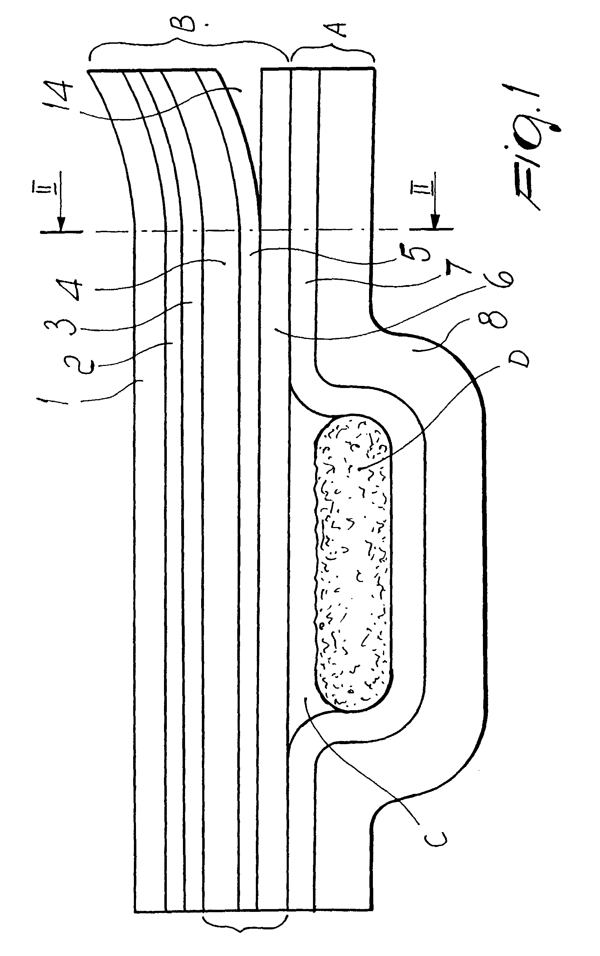

figure 1 is a sectional view of a container according to the invention;
figure 2 is another sectional view, taken along the plane II-II of figure 1.

### Ways of carrying out the Invention

With reference to the above figures, the container is composed of a lower body, designated by the reference letter A, which is closed at the top by a film, generally designated by the reference letter B.

The lower body A forms a tray-like compartment C, in which the volatile product D is to be contained.

The film B is provided by means of a sequence of plies which, starting from the outside, are:
-- a paper ply 1;
-- an aluminum ply 2;
-- a polyester ply 3;
-- a polyethylene ply 4;
-- an EVOH ply 5;
-- a polyethylene ply 6.

The lower body A is obtained by means of a polyethylene ply 7 and a polyacrylonitrile ply 8.

The aluminum ply 2 is substantially the barrier element, while the polyethylene ply 6 is the permeable membrane.

In order to separate the group of plies 1, 2, 3, 4 and 5 from the permeable ply 6, which during use is the element for closing the compartment C, the plies 4, 5 and 6 are first obtained by coextrusion.

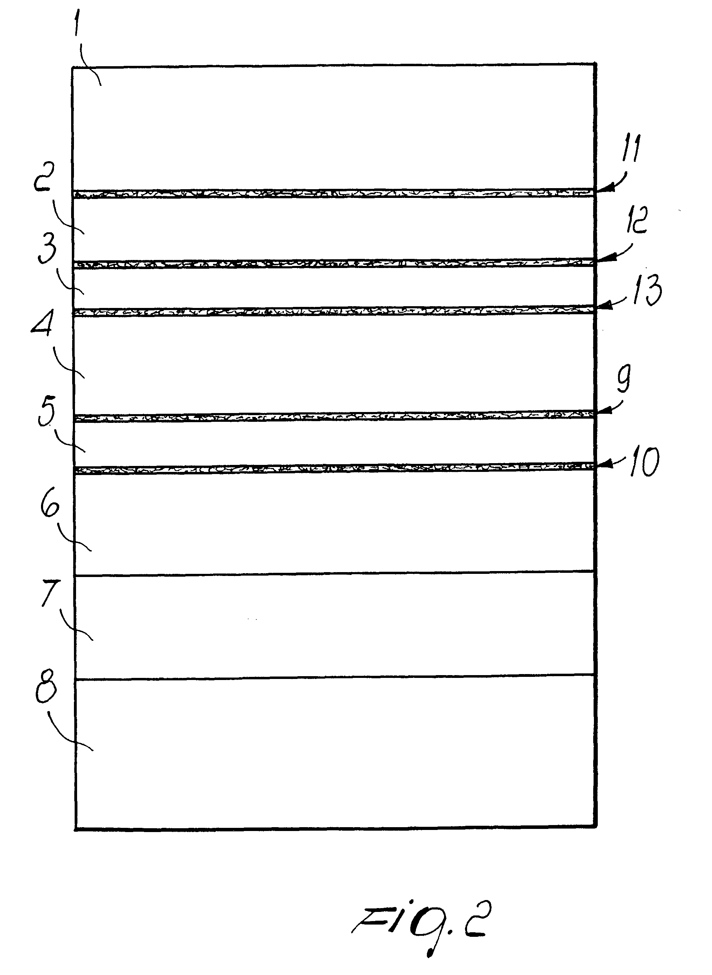

More particularly, as shown in figure 2, coextrusion occurs by interposing a bonding resin 9 between the polyethylene ply 4 and the EVOH ply 5 and a peelable resin 10 between the EVOH ply 5 and the polyethylene ply 6 that constitutes the permeable membrane.

In these conditions, coextrusion is not critical, particularly in terms of temperature, and the bond between the EVOH ply 5 and the polyethylene ply 6 is weaker than the bond between the polyethylene ply 4 and the EVOH ply 5.

The separation region is therefore always located at the peelable resin 10 and the polyethylene ply 6 is in any case not damaged by this operation.

As regards the connection among the other plies, it is convenient to interpose a layer of glue 11 between the paper ply 1 and the aluminum ply 2, while an adhesive 12 is provided between the aluminum ply 2 and the polyethylene ply 3; likewise, a similar adhesive 13 is interposed between the polyester ply 3 and the polyethylene ply 4.

The advantages of a film of this kind are linked to the easy manufacture of the coextruded element, in which it is possible to perfectly control the resistance to ply separation between the EVOH and the polyethylene.

The aluminum barrier, which is known to be much weaker than polyethylene and polyester, is arranged so that it is not affected by mechanical stresses.

From the above description and from the illustrations it is evident that the intended aim and objects have been achieved by adopting a new combination of plies which ensures separation as well as perfect tightness and impermeability to volatile substances when the container is intact.

In order to facilitate separation, a small already-separated region 14 is provided which allows easy grip and guides separation of the plies in the chosen point.

## Claims

1. A container for emanating volatile substances, closed by a film formed by multiple plies including at least one barrier ply for the volatile substance and at least one ply permeable to the volatile substance, characterized in that said film comprises:
- an outer group of plies comprising at least one volatile substance barrier ply; and
- an inner coextruded element coupled on one side to said outer group of plies and on another side to said container, said coextruded element being composed of at least three plies which are, sequentially, a first polyethylene ply, an EVOH ply and a second polyethylene ply, wherein said EVOH ply is detachably coupled to said second polyethylene ply by means of a peelable resin interposed between said EVOH ply and said second polyethylene ply, said second polyethylene ply acting as said at least one volatile substance permeable ply.

2. A container according to claim 1, characterized in that said first polyethylene ply is firmly coupled by means of a bonding resin to said EVOH ply, the coupling between said first polyethylene ply and said EVOH ply being weaker than that between said EVOH ply and said second poltehylene ply which acts as permeable membrane.

3. A container according to claim 2, characterized in that said coextruded element is firmly coupled with the second polyethylene ply side to the container and with the first polyethylene ply to said outer group of plies.

4. A container according to claim 3, characterized in that said outer group of plies is composed externally of a paper ply which is coupled to an aluminum sheet which is in turn coupled to a polyester ply.

5. A container according to claim 1, characterized in that the plies of said multilpe ply film are coupled between then so that the weakest coupling is that between the EVOH ply and the second polyethylene ply.

6. A container according to claim 5, characterized in that the closure of the various plies is hermetic along the edges and constitutes a barrier to the volatile substance.

7. A container according to claim 1, characterized in that it has a small separated region between the EVOH and the permeable membrane which constitutes a grip tab for separating the two parts of the film at this coupling.

## Patentansprüche

1. Behälter zur Abgabe flüchtiger Substanzen, der durch einen aus mehreren Lagen gebildeten Film geschlossen ist und wenigstens eine Sperrlage für die flüchtige Substanz und wenigstens eine für die flüchtige Substanz durchlässige Lage aufweist, **dadurch gekennzeichnet, dass** der Film umfasst:
- eine äußere Gruppe von Lagen mit wenigstens einer Sperrlage für die flüchtige Substanz; und
- ein inneres koextrudiertes Element, das auf einer Seite an die äußere Gruppe von Lagen und auf einer anderen Seite an den Behälter angeschlossen ist und aus wenigstens drei Lagen besteht, die aufeinanderfolgend eine erste Polyethylenlage, eine E/VAL-Lage und eine zweite Polyethylenlage sind, wobei die E/VAL-Lage mittels eines zwischen der E/VAL-Lage und der zweiten Polyethylenlage angeordneten Harzes lösbar mit der zweiten Polyethylenlage verbunden ist und die zweite Polyethylenlage als die wenigstens eine für die flüchtige Substanz durchlässige Lage fungiert.

2. Behälter nach Anspruch 1, **dadurch gekennzeichnet, dass** die erste Polyethylenlage fest mit der E/VAL-Lage verbunden ist, wobei die Verbindung zwischen der ersten Polyethylenlage und der E/VAL-Lage schwächer als die zwischen der E/VAL-Lage und der zweiten, als durchlässige Membran fungierenden Polyethylenlage ist.

3. Behälter nach Anspruch 2, **dadurch gekennzeichnet, dass** das koextrudierte Element auf der Seite der zweiten Polyethylenlage fest mit dem Behälter und mit der ersten Polyethylenlage mit der äußeren Gruppe von Lagen verbunden ist.

4. Behälter nach Anspruch 3, **dadurch gekennzeichnet, dass** die äußere Gruppe von Lagen außen aus einer Papierlage besteht, die mit einem Aluminiumblatt verbunden ist, das seinerseits mit einer Polyesterlage verbunden ist.

5. Behälter nach Anspruch 1, **dadurch gekennzeichnet, dass** die Lagen des aus mehreren Lagen bestehenden Filmes miteinander so verbunden sind, dass die schwächste Verbindung die zwischen der E/VAL-Lage und der zweiten Polyethylenlage ist.

6. Behälter nach Anspruch 5, **dadurch gekennzeichnet, dass** der Verschluss der verschiedenen Lagen entlang den Kanten hermetisch ist und eine Sperre für die flüchtige Substanz bildet.

7. Behälter nach Anspruch 1, **dadurch gekennzeichnet, dass** er einen kleinen separierten Bereich zwischen der E/VAL- und der durchlässigen Membran aufweist, der eine Grifflasche zur Trennung der beiden Teile des Films an dieser Verbindung bildet.

## Revendications

1. Un conteneur pour l'évaporation des substances volatiles, fermé par une feuille formée de multiples couches incluant au moins une couche de barrage aux substances volatiles et au moins une couche perméable aux substances volatiles, caractérisé en ce que ladite feuille comprend :
- un groupe externe de couches comprenant au moins une couche de barrage aux substances volatiles ; et
- un élément co-extrudé interne couplé sur un côté audit groupe de couches externes et sur un autre côté du dit conteneur, ledit élément co-extrudé étant composé d'un moins trois couches qui sont, séquentiellement, une première couche en polyéthylène, une couche en EVOH et une seconde couche en polyéthylène, dans lequel ladite couche en EVOH est couplée de façon détachable à ladite seconde couche en polyéthylène au moyen d'une résine pelable intercalée entre ladite couche en EVOH et ladite seconde couche en polyéthylène, ladite seconde couche en polyéthylène agissant comme ladite au moins une couche perméable aux substances volatiles.

2. Un conteneur selon la revendication 1, caractérisé en ce que ladite première couche en polyéthylène est fermement couplée au moyen d'une résine de liaison à ladite couche en EVOH, le couplage entre ladite première couche en polyéthylène et ladite couche en EVOH étant plus faible que celle entre ladite couche en EVOH et ladite seconde couche en polyéthylène qui agit comme une membrane perméable.

3. Un conteneur selon la revendication 2, caractérisé en ce que ledit élément co-extrudé est fermement couplé au conteneur sur un côté de la seconde couche en polyéthylène et à l'aide de la première couche en polyéthylène audit groupe de couches externes.

4. Un conteneur selon la revendication 3, caractérisé en ce que le groupe de couches externes est composé d'une façon externe d'une couche en papier qui est couplé à une couche d'aluminium qui est à son tour couplée à une couche en polyester.

5. Un conteneur selon la revendication 1, caractérisé en ce que les couches de ladite feuille de couches multiples sont couplées entre elles de telle façon que le couplage le plus faible est celui entre la couche en EVOH et la seconde couche en polyéthylène.

6. Un conteneur selon la revendication 5, caractérisé en ce que la fermeture des couches variées est hermétique le long des côtés et constitue une barrière à la substance volatile.

7. Un conteneur selon la revendication 1, caractérisé en ce qu'il présente une petite zone séparée entre la membrane en EVOH et la membrane perméable qui constitue une attache pour séparer les deux parties de la feuille au niveau de ce couplage.
